# EUROPEAN PATENT SPECIFICATION

(11) **EP 3 870 255 B1**
(45) Date of publication and mention of the grant of the patent: **11.12.2024**
(21) Application number: 19790224.0
(22) Date of filing: 25.10.2019
(51) Int. Cl.: A61M 5/19, A61M 5/20, A61M 5/31, A61M 5/315

(54) **ZERO-POINT ADJUSTMENT OF PREFILLED DRUG DELIVERY DEVICE**
NULLPUNKTEINSTELLUNG EINER VORGEFÜLLTEN ARZNEIMITTELABGABEVORRICHTUNG
RÉGLAGE DU POINT ZÉRO D'UN DISPOSITIF D'ADMINISTRATION DE MÉDICAMENTS PRÉREMPLIS

(30) Priority: 26.10.2018 EP 18202896
(43) Date of publication of application: 01.09.2021
(73) Proprietor: Novo Nordisk A/S, 2880 Bagsværd (DK)
(72) Inventor: JENSEN, Nicolai, Michael, 2880 Bagsværd (DK); BENDIX, Klaus, 2880 Bagsværd (DK)
(86) International application number: PCT/EP2019/079155
(87) International publication number: WO 2020/084109

(56) References cited:
- US-A1- 2010 280 461
- US-A1- 2011 046 567
- US-A1- 2016 206 822

## Description

### FIELD OF THE INVENTION

The present invention relates generally to drug delivery devices, and more specifically to zero-point adjustment of prefilled injection devices, including twin chamber devices having a single dose engine.

### BACKGROUND OF THE INVENTION

Today, many devices for self-administration of liquid drugs are based on technologies for percutaneous delivery, e.g. injection devices or infusion pumps, or for pulmonary delivery, e.g. inhalers. In the art of such drug delivery devices there is a marked distinction between a so-called prefilled device and a so-called reusable device. The term "reusable device" designates a drug delivery device which employs a user exchangeable drug container, whereas the term "prefilled device" designates a drug delivery device which carries a non-exchangeable drug container that is pre-mounted by the manufacturer. Hence, where the former is adapted to be used for exhaustion of multiple drug containers, the latter is meant to be discarded after emptying of the pre-mounted drug container.

In large scale manufacturing and assembly of prefilled drug delivery devices, such as prefilled injection pens commonly used in the diabetes care segment for administration of e.g. insulin or glp-1, the tolerance chains are likely to result in relative positions of specific internal components varying slightly from device to device. For example, when a drug containing cartridge comprising a slidable piston is attached to a pen housing the piston may not perfectly align with a piston rod in the pen housing adapted to thrust the piston forward in the cartridge for expelling of the drug. Consequently, an undesired clearance between the piston and the piston rod may be established which needs to be eliminated to ensure dose predictability by the user performing an initial priming action before the first dose administration.

The initial priming action consists of the user setting a small dose and activating the delivery mechanism to expel the set dose. The piston rod will thereby be urged distally a predetermined distance by the piston rod. The predetermined distance travelled by the piston rod will be sufficient to eliminate any initial clearance between the piston rod and the piston but will also cause a small amount of drug to be discharged from the cartridge.

So, not only is initial priming of a prefilled drug delivery device an additional activity which is imposed on the user it also inevitably leads to some wastage of drug. This wastage of drug may be pronounced for drug delivery devices of the fixed dose type, which typically only allow for setting of a particular predetermined dose. It is therefore highly desirable to provide a prefilled drug delivery device in which the piston and the piston actuator are always in physical contact such that initial priming is unnecessary.

WO 2017/072233 (Novo Nordisk A/S) discloses a method of manufacturing prefilled drug delivery devices and teaches how to avoid initial priming by performing a dedicated zero-point adjustment of the drug delivery devices during manufacturing. The zero-point adjustment comprises using a piston of a drug reservoir to move a piston rod proximally in a nut member of a device housing from an initial assembly position to a final assembly position and subsequently preventing further proximal motion of the piston rod by rotationally restricting a piston rod drive member. The prevention of further proximal motion of the piston rod is a prerequisite for eliminating the risk of subsequently introducing an air gap between the piston rod and the piston.

The disclosed method requires operations on the piston rod from the distal end of the device housing as well as operations on the piston rod drive member from the proximal end of the device housing and may, accordingly, in some cases be viewed as cumbersome. Hence, it is desirable to provide a solution which facilitates zero-point adjustment of prefilled drug delivery devices in the production line.

US 2010/0280461, US 2011/0046567, and US 2016/0206822 are further examples of approaches to eliminating clearance between a piston rod and a piston, where US 2010/0280461 discloses an adjusting member arranged between a piston rod and a piston, US 2011/0046567 discloses an axially adjustable connection between a piston rod and a piston rod foot, and US 2016/0206822 discloses a two-part piston rod having an adjustable length.

Within some medical treatment areas, a combination therapy involving co-administration of at least two active agents is advantageous because of synergistic or additive effects. For example, within diabetes care, in the management of type 2 diabetes mellitus, concomitant use of certain insulin and glp-1 products has been shown to reduce HbA_{1c} levels in subjects, thereby improving glycaemic control.

Percutaneous drug delivery is often associated with discomfort as many people dislike the thought of having an injection needle inserted through the skin. An undisclosed number of people even suffer from needle-phobia. It is therefore an attractive scenario to reduce the number of required skin penetrations by administering the injectable media at the same time, or substantially the same time. In that respect prefabricated mixtures of the involved media are not always an optimal solution. For one, some substances are only stable in mixed form short-term, and it may accordingly be necessary to keep those substances apart until just prior to administration. Adding to that, the individual subject users may have different needs in terms of dose ratios of the constituent active ingredients. Even a single subject user may sometimes require varying dose ratios of the active ingredients over a relatively short time span, e.g. during a titration period. It may thus not be feasible to cover all the individual needs by premixed pharmaceutical products.

Different solutions exist which do not employ prefabricated mixtures. Dual chamber drug delivery devices typically carry a single elongated cartridge having two spaced apart pistons, establishing a front chamber for the one medium and a rear chamber for the other medium, and bypass means to allow for passage of the medium in the rear chamber when the separating piston is in a particular position or state. Such devices allow for administration of the two media substantially simultaneously or sequentially via a single outlet interface, thus avoiding the need for two separate needle pricks. An example of a dual chamber drug delivery device is disclosed in US 4,394,863 (Survival Technology, Inc.).

Presenting an alternative configuration, twin chamber drug delivery devices keep the two media separated in parallel drug reservoirs, the two reservoir outlets being fluidly connected by a manifold needle unit with a single injection needle. The reservoirs are typically individually pressurised by dedicated actuators, which may either be operated by separate dose engines or by a single, common dose engine. An example of the latter is disclosed in WO 2017/114921 (Novo Nordisk A/S).

For twin chamber drug delivery devices with a single dose engine where the two actuators are operatively coupled it can be a challenge during manufacturing to perform a zero-point adjustment of the dosing system since movement of the one actuator affects the position of the other. As it is crucial for the dosing accuracy of the drug delivery device that each actuator is in contact with its associated drug reservoir at the time of activation of the dose expelling mechanism it is desirable to provide a solution which facilitates zero-point adjustment of such devices in the production line.

### SUMMARY OF THE INVENTION

It is an object of the invention to eliminate or reduce at least one drawback of the prior art, or to provide a useful alternative to prior art solutions.

In particular, it is an object of the invention to provide a drug delivery device solution which enables a simple and easy to perform zero-point adjustment.

It is a further object of the invention to provide a method for performing zero-point adjustment of a drug delivery device, which is simple and easy carry out.

It is an even further object of the invention to provide a solution which allows for simple and easy zero-point adjustment of a twin chamber drug delivery device with a single dose engine.

In the disclosure of the present invention, aspects and embodiments will be described which will address one or more of the above objects and/or which will address objects apparent from the following text.

In a first aspect of the invention a drug delivery device as specified in claim 1 is provided.

Accordingly, a drug delivery device is provided which comprises a cartridge unit comprising a cartridge holder carrying a drug cartridge having a cartridge body and a piston structure, and a dose expelling unit comprising a housing and a piston rod for pressurising the drug cartridge by interaction with the piston structure, where the piston rod is engaged with a nut member in a non-self-locking thread engagement and is rotationally interlocked with a piston rod drive member. The piston rod drive member is initially bidirectionally rotatable about a longitudinal axis such that a rotation of the piston rod drive member in a first direction causes distal movement of the piston rod through the nut member, and vice versa, and a rotation of the piston rod drive member in a second direction, opposite to the first direction, causes proximal movement of the piston rod through the nut member, and vice versa.

The nut member is rotationally fixed, but initially axially displaceable, with respect to the housing, and the piston rod drive member is axially fixed with respect to the housing. The nut member comprises a first coupling part of a unidirectional ratchet coupling and the piston rod drive member is operatively coupled with a second coupling part of the unidirectional ratchet coupling, and the nut member is initially axially displaceable relative to the housing and the piston rod drive member from a first nut member position in which said first coupling part and said second coupling part are disengaged, providing for the piston rod drive member being initially bidirectionally rotatable, and a second nut member position in which said first coupling part and said second coupling part engage to prevent rotation of the piston rod drive member in the second direction and, accordingly, further proximal displacement of the piston rod.

The nut member is brought irreversibly from the first nut member position to the second nut member position during assembly of the dose expelling unit and the cartridge unit, which involves a bringing together of the housing and the cartridge holder along the longitudinal axis, after contact between the piston structure and the piston rod is established. The construction of the housing with the initially axially displaceable nut thus allows for an easy elimination of any air gaps between the piston rod and the piston structure during manufacturing of the drug delivery device in that a simple converging relative axial motion between the cartridge holder and the housing, carried out during assembly of the cartridge unit and the dose expelling unit after the piston structure and the piston rod are brought into abutment, firstly allows the piston structure to urge a proximal motion of the piston rod through the non-self-locking thread and secondly prompts e.g. the cartridge holder to abut and move the nut member from the first nut member position to the second nut member position, thereby establishing a ratchet connection between the nut member and the piston rod drive member which prevents the piston rod from further proximal motion through the non-self-locking thread and resultantly ensures continued abutment between the piston rod and the piston structure.

The contact between the piston structure and the contact surface may be established during an initial converging relative axial motion between the cartridge holder and the housing, and the nut member may be brought from the first nut member position to the second nut member position during a subsequent converging relative axial motion between the cartridge unit and the housing. The zero-point adjustment as well as the securing of the piston rod may thus be carried out entirely by simple relative axial motion between the cartridge holder and the housing.

In an exemplary embodiment of the invention a proximal rim portion of the cartridge holder is structured to apply a proximally directed force to at least one distally projecting leg of the nut member during the converging relative axial motion between the housing and the cartridge holder, and the proximally directed force brings the nut member from the first nut member position to the second nut member position.

The cartridge unit may further comprise a second drug cartridge having a second cartridge body and a second piston structure, where the drug cartridge and the second drug cartridge are held in parallel by the cartridge holder. Accordingly, the dose expelling unit may further comprise a second piston rod for pressurising the second drug cartridge, the second piston rod comprising a second contact surface adapted to contact the second piston structure, and a second piston rod thread in engagement with a second thread of the nut member, and a second piston rod drive member axially locked with respect to the housing and initially bidirectionally rotatable about a second axis parallel with the longitudinal axis, the second piston rod drive member and the second piston rod being rotationally interlocked, and the second piston rod drive member being rotationally coupled with the piston rod drive member. In such cases the second piston rod has an axially variable dimension which is set during assembly of the dose expelling unit and the cartridge unit, after contact between the second piston structure and the second piston rod is established. In particular, the second piston rod may comprise a telescopic structure capable of being transformed to a functionally unitary structure to thereby set the axially variable dimension. For example, the second piston rod may comprise a front piston rod portion and a rear piston rod portion which are initially capable of undergoing relative sliding motion along one another and which are axially interlocked during assembly of the dose expelling unit and the cartridge unit, after contact between the second piston structure and the second piston rod is established.

Thereby, a solution for eliminating any air gaps between any of the piston and the piston rod and the second piston and the second piston rod in a twin chamber drug delivery device is provided. The air gap elimination may be ensured during assembly of the cartridge unit and the dose expelling unit by simply inducing a converging relative axial motion between the cartridge holder and the housing, just as described above. The two-part second piston rod allows for establishment of contact between both piston structure/piston rod pairs even though the two piston rod drive members are rotationally coupled because it is initially capable of changing axial extent. Once said contact is established the front piston rod portion and the rear piston rod portion are axially secured to one another, rendering the second piston rod axially rigid and capable of transferring forces to the second piston for expelling of drug from the second drug cartridge. The front piston rod portion and the rear piston rod portion may for example be axially interlocked by laser welding through a bore in the cartridge holder.

The piston rod drive member and the second piston rod drive member may be arranged in a casing which is fixed to the housing. The casing may be provided with a distal slot and a proximal slot axially spaced apart from the distal slot, and the nut member may comprise a protrusion adapted to move from a position in the distal slot to a position in the proximal slot during movement of the nut member from the first nut member position to the second nut member position and structured to prevent subsequent movement of the nut member towards the first nut member position. The nut member will thereby become axially fixed with respect to the housing when it reaches the second nut member position, preventing subsequent disengagement of the first coupling part of the unidirectional ratchet coupling from the second coupling part of the unidirectional ratchet coupling.

The drug delivery device may further comprise a central gearwheel, the piston rod drive member may comprise a first gearwheel toothing and the second piston rod drive member may comprise a second gearwheel toothing, and the piston rod drive member and the second piston rod drive member may be rotationally coupled via the central gearwheel. This provides a compact drive mechanism for the twin chamber drug delivery device. Particularly, the second coupling part may form part of the central gearwheel.

The second piston rod thread may be self-locking, or may at least have a pitch which is smaller than that of the non-self-locking thread. The two piston rods are thereby in a geared relationship, wherein the second piston rod is displaced axially less than the piston rod during (the equal) rotation of the piston rod driver and the second piston rod driver. An exemplary gearing ratio is 3:1.

The drug cartridge may further comprise a drug outlet sealed by a penetrable septum, and the second drug cartridge may further comprise a second drug outlet sealed by a second penetrable septum. When the penetrable septum and the second penetrable septum are axially aligned relative to the cartridge holder the second piston structure may be positioned distally of the piston structure. This may for example be the case if the axial dimension of the second drug cartridge is smaller than the axial dimension of the drug cartridge.

In a second aspect the invention provides a method of performing zero-point adjustment of a drug delivery device comprising A) a cartridge unit comprising a cartridge holder carrying a drug cartridge having a cartridge body and a piston structure, and a dose expelling unit comprising a housing extending along a longitudinal axis, a piston rod for pressurising the drug cartridge, the piston rod comprising a contact surface adapted to contact the piston structure, and a non-self-locking thread, a nut member rotationally fixed with respect to the housing and engaged with the non-self-locking thread, the nut member carrying a first coupling part of a unidirectional ratchet coupling, and a piston rod drive member axially fixed with respect to the housing and rotatable about the longitudinal axis, the piston rod drive member being operatively coupled with a second coupling part of the unidirectional ratchet coupling, wherein the piston rod drive member and the piston rod are rotationally interlocked, such that a rotation of the piston rod drive member in a first direction is associated with distal helical movement of the piston rod in the nut member, and a rotation of the piston rod drive member in a second direction is associated with proximal helical movement of the piston rod in the nut member.

The method comprises: (i) arranging the piston rod in the nut member such that the contact surface is positioned distally of a final contact surface assembly position, (ii) bringing the piston structure and the contact surface into mutual abutment, (iii) inducing a converging relative axial motion between the cartridge holder and the housing, thereby firstly forcing the piston rod proximally in the nut member by means of the piston structure and secondly moving the nut member axially relative to the housing from a first nut member position in which the first coupling part and the second coupling part are disengaged to a second nut member position in which the first coupling part and the second coupling part are engaged, rendering the piston rod drive member unidirectionally rotatable in the first direction, and (iv) axially interlocking the cartridge holder and the housing.

The method provides for zero-point adjustment, i.e. elimination of any slack between the piston rod and the piston, by a simple converging relative axial motion between the cartridge holder and the housing which is easily carried out during manufacturing of the drug delivery device in connection with the joining of the cartridge unit and the dosing unit. Hence, by this method there is no need to perform operations on the piston rod drive member from the proximal end of the device housing, as is for example the case in WO 2017/072233.

In an exemplary embodiment of the invention bringing the piston structure and the contact surface into mutual abutment involves inducing a converging relative axial motion between the cartridge holder and the housing. The entire final assembly of the cartridge unit and the dosing unit, including the zero-point adjustment, can thus be executed by mere translational motion of one of the cartridge holder and the housing towards the other or by mere translational motion of the cartridge holder and the housing towards one another.

In a third aspect the invention provides a method of performing zero-point adjustment of a drug delivery device comprising A) a cartridge unit comprising a cartridge holder carrying a1) a first drug cartridge having a first cartridge body and a first piston structure, and a2) a second drug cartridge having a second cartridge body and a second piston structure, and B) a dose expelling unit comprising b1) a housing extending along a longitudinal central axis, b2) a first piston rod for pressurising the first drug cartridge, the first piston rod comprising a first contact surface adapted to contact the first piston structure, and a non-self-locking thread, b3) a second piston rod for pressurising the second drug cartridge, the second piston rod comprising a front piston rod portion having a second contact surface adapted to contact the second piston structure, and a rear rod portion having a second piston rod thread, the front piston rod portion and the rear piston rod portion being capable of undergoing relative sliding motion along one another, b4) a nut member rotationally fixed with respect to the housing and respectively engaged with the non-self-locking thread and the second piston rod thread, the nut member carrying a first coupling part of a unidirectional ratchet coupling, b5) a first piston rod drive member axially fixed with respect to the housing and rotatable about a first axis parallel with the longitudinal central axis, the first piston rod drive member being operatively coupled with a second coupling part of the unidirectional ratchet coupling, and the first piston rod drive member and the first piston rod being rotationally interlocked such that a rotation of the first piston rod drive member in a first direction about the first axis is associated with distal helical movement of the first piston rod in the nut member, and a rotation of the first piston rod drive member in a second direction about the first axis is associated with proximal helical movement of the first piston rod in the nut member, and b6) a second piston rod drive member axially fixed with respect to the housing and rotatable about a second axis parallel with the longitudinal central axis, the second piston rod drive member being rotationally coupled with the first piston rod drive member, and the second piston rod drive member and the rear piston rod portion being rotationally interlocked such that a rotation of the second piston rod drive member in a first direction about the second axis is associated with distal helical movement of the rear piston rod portion in the nut member, and a rotation of the second piston rod drive member in a second direction about the second axis is associated with proximal helical movement of the rear piston rod portion in the nut member. The method comprises: (i) arranging the first piston rod and the second piston rod in the nut member such that the first contact surface is positioned distally of a final first contact surface assembly position and the second contact surface is positioned distally of a final second contact surface assembly position, (ii) aligning the cartridge holder and the housing along the longitudinal central axis such that the first piston structure is aligned with the first axis and the second piston structure is aligned with the second axis, (iii) inducing a converging relative axial motion between the cartridge holder and the housing, said motion comprising a first part motion which brings the cartridge holder and the housing to an intermediate assembly position and in the course of which (but not necessarily during the entire first part motion) the first contact surface is moved proximally relative to the nut member by means of the first piston structure and the second contact surface is moved proximally relative to the rear piston rod portion, the second piston rod thereby obtaining an intermediate second piston rod assembly configuration, and a second part motion which brings the cartridge holder and the housing to a final assembly position and during which the first piston rod, the second piston rod, and the nut member are moved jointly relative to the first piston rod drive member, the first coupling part of the unidirectional ratchet coupling thereby entering into engagement with the second coupling part of the unidirectional ratchet coupling, rendering the first piston rod drive member unidirectionally rotatable in the first direction about the first axis and the second piston rod drive member unidirectionally rotatable in the first direction about the second axis, (iv) axially interlocking the front piston rod portion and the rear piston rod portion in the intermediate second piston rod assembly configuration, and (v) axially interlocking the cartridge holder and the housing in the final assembly position.

The method provides for zero-point adjustment of a twin chamber drug delivery device with a single dose engine, i.e. elimination of any slack between the respective piston rods and pistons, by a simple converging relative axial motion between the cartridge holder and the housing which is easily carried out during manufacturing of the drug delivery device in connection with the joining of the cartridge unit and the dosing unit.

The possibility of changing the axial extent of the second piston rod is advantageous because it allows for a relatively course arrangement of the first piston rod and the second piston rod in step (i). It is desirable to avoid having to precisely position the first piston rod and the second piston rod in the nut member for each dose expelling unit in a batch in order to accommodate various manufacturing tolerances, e.g. affecting the positions of the first piston structure and the second piston structure with respect to the cartridge holder which the respective dose expelling unit is to be mated with, to ensure that contact between the first contact surface and the first piston structure, respectively the second contact surface and the second piston structure is established simultaneously during step (iii).

In embodiments where the pitch of the non-self-locking thread is different from the pitch of second piston rod thread the first piston rod and the rear piston rod portion move different axial distances when the rotationally coupled first piston rod drive member and second piston rod drive member undergo the same rotation. The variability of the axial dimension of the second piston rod enables an elimination of air gaps in both the first piston rod/piston structure system and the second piston rod/piston structure system, also for such embodiments, because it allows axial motion of the second contact surface independently of both the first contact surface and the rear piston rod portion. The respective contacts can thus be established even though they are not established simultaneously and even though the first piston rod drive member and the second piston rod drive member are rotationally coupled and accordingly rotate together.

In a fourth aspect the invention provides a drug delivery device comprising: a cartridge unit comprising a cartridge holder, a first cartridge having a first cartridge body and a first piston, and a second cartridge having a second cartridge body and a second piston, the first cartridge and the second cartridge being held in parallel by the cartridge holder, and a dose expelling unit comprising a housing, a first expelling system for expressing a first liquid substance from the first cartridge, the first expelling system comprising a first piston rod for advancing the first piston in the first cartridge body, the first piston rod being in non-self-locking threaded engagement with a first thread segment rotationally fixed with respect to the housing, and a first piston rod drive member rotationally fixed with respect to the first piston rod and rotatable about a first axis to advance the first piston rod along the first axis, a second expelling system for expressing a second liquid substance from the second cartridge, the second expelling system comprising a second piston rod for advancing the second piston in the second cartridge body, the second piston rod being in threaded engagement with a second thread segment rotationally fixed with respect to the housing, and a second piston rod drive member rotationally fixed with respect to the second piston rod and rotatable about a second axis to advance the second piston rod along the second axis, the second piston rod drive member further being rotationally coupled with the first piston rod drive member, wherein the second piston rod has an extension along the second axis which is initially variable and which is determined and fixed during assembly of the dose expelling unit and the cartridge unit, after contact between the second piston and the second piston rod is established.

Thereby, a drug delivery device is provided which comprises a united dose expelling unit and cartridge unit, where the cartridge unit carries a first cartridge which comprises a first cartridge body and a first piston and holds a first liquid substance, and a second cartridge which comprises a second cartridge body and a second piston and holds a second liquid substance, and where the dose expelling unit comprises a housing accommodating, at least partially, a first dose expelling system and a second dose expelling system.

The first cartridge and the second cartridge are held in parallel by the cartridge holder, e.g. arranged in parallel within the cartridge holder, and the first dose expelling system is configured to expel a dose of the first liquid substance from the first cartridge, while the second dose expelling system is configured to expel a dose of the second liquid substance from the second cartridge.

The first dose expelling system comprises a first piston rod for advancing the first piston in the first cartridge body and a first piston rod drive member coupled with the first piston rod and adapted to rotate about a first axis to advance the first piston rod along the first axis. The first piston rod comprises a first piston rod thread, which is non-self-locking and which engages a first thread segment being rotationally fixed with respect to the housing, e.g. a first nut structure of a nut member arranged in the housing.

The second dose expelling system comprises a second piston rod for advancing the second piston in the second cartridge body and a second piston rod drive member coupled with the second piston rod and adapted to rotate about a second axis to advance the second piston rod along the second axis. The second piston rod comprises a second piston rod thread engaging a second thread segment being rotationally fixed with respect to the housing, e.g. a second nut structure of the nut member. The second piston rod thread may be self-locking or non-self-locking.

The first piston rod drive member and the second piston rod drive member are rotationally coupled, i.e. a rotation in either direction of the one causes a reactive rotation of the other. Thereby, a single rotational input motion is sufficient to actuate both piston rod drive members.

The second piston rod has an extension along the second axis which is initially variable and which is determined and rendered non-variable during assembly of the dose expelling unit and the cartridge unit, which involves a bringing together of the housing and the cartridge holder along a longitudinal axis parallel to the first axis and the second axis, after contact between the second piston and the second piston rod is established.

For example, the second piston rod may comprise a front piston rod portion and a rear piston rod portion which are initially capable of undergoing relative sliding motion along one another and which are interlocked, after contact between the second piston and the second piston rod is established.

Such a construction allows for easy zero-point adjustment of a twin chamber drug delivery device with a single dose engine by mere converging relative axial motion between the housing and the cartridge holder. The respective initial axial positions of the first piston rod and the second piston rod in the housing may, intentionally or unintentionally, differ, as may the respective initial axial positions of the first piston and the second piston in the cartridge holder.

Even though the two piston rod drive members are rotationally coupled, and a rotation (with accompanying proximal displacement) of the one piston rod thus causes a rotation (with accompanying proximal displacement) of the other, the fact that the extension of the second piston rod is initially variable enables initial asynchronous movement of the respective distal end faces of the first piston rod and the second piston rod. This means that regardless of which piston is brought into contact with its associated piston rod first during an initial phase of the converging relative axial motion between the housing and the cartridge holder the distal end face of the piston rod in question may simply be displaced proximally with respect to the housing without causing movement of the distal end face of the other piston rod, thereby allowing the other piston to subsequently be brought into contact with its associated piston rod.

Specifically, if the first piston is brought into contact with the first piston rod before the second piston reaches the second piston rod the first piston rod will during the continued converging relative axial motion between the housing and the cartridge holder be pushed backwards through the first thread segment (which is possible due to the non-self-locking threaded engagement), thereby causing a backwards displacement of the rear piston rod portion of the second piston rod due to the rotational coupling between the first piston rod drive member and the second piston rod drive member. However, gravity and/or a frictionless interface between the front piston rod portion and the rear piston rod portion allows the front piston rod portion to remain in place, thereby increasing the axial extension of the second piston rod until contact is established between the approaching second piston and a distal end face of the front piston rod portion, at which point both dose expelling systems are primed.

Alternatively, if the second piston is brought into contact with the second piston rod before the first piston reaches the first piston rod the front piston rod portion will during the continued converging relative axial motion between the housing and the cartridge holder slide backwards relative to the rear piston rod portion which remains stationary in the second thread segment, thereby decreasing the axial extension of the second piston rod without causing movement of the first piston rod. At some point during this movement of the front piston rod portion relative to the rear piston rod portion the first piston will reach the first piston rod, and both dose expelling systems will then be primed.

In a fifth aspect the invention provides a method of positioning a first piston rod and a second piston rod relative to a housing of a drug delivery device, the first piston rod being in non-self-locking threaded connection with a first thread segment rotationally fixed with respect to the housing and the second piston rod being in threaded connection with a second thread segment rotationally fixed with respect to the housing, the first piston rod further being rotationally locked with respect to a first piston rod drive member which is axially fixed with respect to the housing and initially bidirectionally rotatable about a first longitudinal axis, the second piston rod further comprising a front piston rod portion and a rear piston rod portion capable of axial displacement relative to one another, at least the rear piston rod portion being rotationally locked with respect to a second piston rod drive member which is axially fixed with respect to the housing and initially bidirectionally rotatable about a second longitudinal axis, and the first piston rod drive member and the second piston rod drive member being rotationally coupled via a rotatable clutch, where rotation of the rotatable clutch in a first direction is associated with forward displacement of the first piston rod and the rear piston rod portion and rotation of the rotatable clutch in a second direction is associated with backward displacement of the first piston rod and the rear piston rod portion, the method comprising: (i) placing the first piston rod in an initial first piston rod assembly position and the second piston rod in an initial second piston rod assembly position relative to the housing, (ii) aligning a cartridge holder and the housing axially, the cartridge holder carrying a first axially extending prefilled cartridge sealed by a first penetrable septum and a first piston and a second axially extending prefilled cartridge sealed by a second penetrable septum and a second piston, (iii) inducing a converging relative translational motion between the cartridge holder and the housing, said converging relative translational motion comprising a first part motion which brings the cartridge holder and the housing to an intermediate assembly position and during which the first piston moves the first piston rod relative to the first thread segment to an intermediate first piston rod assembly position and the second piston moves the front piston rod portion relative to the rear piston rod portion, the second piston rod thereby reaching an intermediate second piston rod assembly position, and a second part motion which brings the cartridge holder and the housing to a final assembly position and during which the first piston, the first piston rod and the first thread segment move jointly relative to the first piston rod drive member, the first piston rod thereby reaching a final first piston rod assembly position, and the second piston, the front piston rod portion, the rear piston rod portion and the second tread segment move jointly relative to the second piston rod drive member, the second piston rod thereby reaching a final second piston rod assembly position, (iv) axially interlocking the front piston rod portion and the rear piston rod portion in the intermediate second piston rod assembly position or in the final second piston rod assembly position, and (v) axially interlocking the cartridge holder and the housing in the final assembly position.

Step (iv) may be carried out by laser welding, gluing or otherwise immobilising the front piston rod portion relative to the rear piston rod portion so as to render the second piston rod axially rigid.

Step (v) may be carried out by simply snap fitting the cartridge holder and the housing or, alternatively, by laser welding, gluing, or other suitable means.

The first thread segment and the second thread segment may form part of a nut member and the nut member may comprise a first coupling part of a unidirectional ratchet coupling. The rotatable clutch may comprise a second coupling part of the unidirectional ratchet coupling, and the second part motion may bring the first coupling part and the second coupling part into disengageable engagement, thereby preventing rotation of the rotatable clutch in the second direction. Thereby, it is ensured that once the first piston rod is brought to the final first piston rod assembly position and the second piston rod is brought to the final second piston rod assembly position the first piston rod can no longer move proximally, or backwards, through the first thread segment and the second piston rod can no longer move proximally, or backwards, through the second thread segment. The contact established between the respective piston rods and pistons is thus sustained throughout the use of the drug delivery device.

A drug cartridge, or simply a cartridge, is typically a reservoir of the type conventionally used in pen injection devices, i.e. comprising a generally cylindrical cartridge body with a neck portion, which cartridge body is sealed by, respectively, a piston and a penetrable self-sealing septum and accommodates a volume of drug.

It is noted that in the present context the term "piston rod" can encompass both a piston rod as such and a piston rod structure comprising a piston rod in combination with a piston washer or piston rod foot. Similarly, the term "piston" can encompass both a piston as such and a piston structure comprising a piston in combination with a piston washer. Specifically, during manufacturing of a drug delivery device according to the invention a present piston washer may initially be connected to a piston rod or to a piston. Regardless of which, the zero-point adjustment leads to establishment of a contact between the piston rod and the piston which in case a piston washer is present is rather an operational contact via the piston washer in the sense that the physical contact is obtained between the piston rod and the piston washer, respectively between the piston washer and the piston.

Further, as used herein, the terms "distal" and "proximal" denote positions at or directions along a drug delivery device, where "distal" refers to the drug outlet end and "proximal" refers to the end opposite the drug outlet end.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context. The invention is defined by the appended claims.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 is an exploded view of a reservoir sub-assembly and a piston rod sub-assembly of a drug delivery device according to an embodiment of the invention,
Fig. 2 is a longitudinal section view of the reservoir sub-assembly and the piston rod sub-assembly,
Figs. 3-6 are longitudinal section views of the reservoir sub-assembly and the piston rod sub-assembly in respective states during a zero-point adjustment procedure according to an embodiment of the invention,
Figs. 7a and 7b are partially sectioned, perspective views of a nut member and a transmission case in different assembly states,
Figs. 8a and 8b are side-views of the different assembly states of Figs. 7a and 7b, and
Fig. 9 is a longitudinal section view of an assembled drug delivery device.

In the figures like structures are mainly identified by like reference numerals.

### DESCRIPTION OF EXEMPLARY EMBODIMENTS

When/If relative expressions, such as "upper" and "lower", "left" and "right", "horizontal" and "vertical", "clockwise" and "counter-clockwise", etc., are used in the following, these refer to the appended figures and not necessarily to an actual situation of use. The shown figures are schematic representations for which reason the configuration of the different structures as well as their relative dimensions are intended to serve illustrative purposes only.

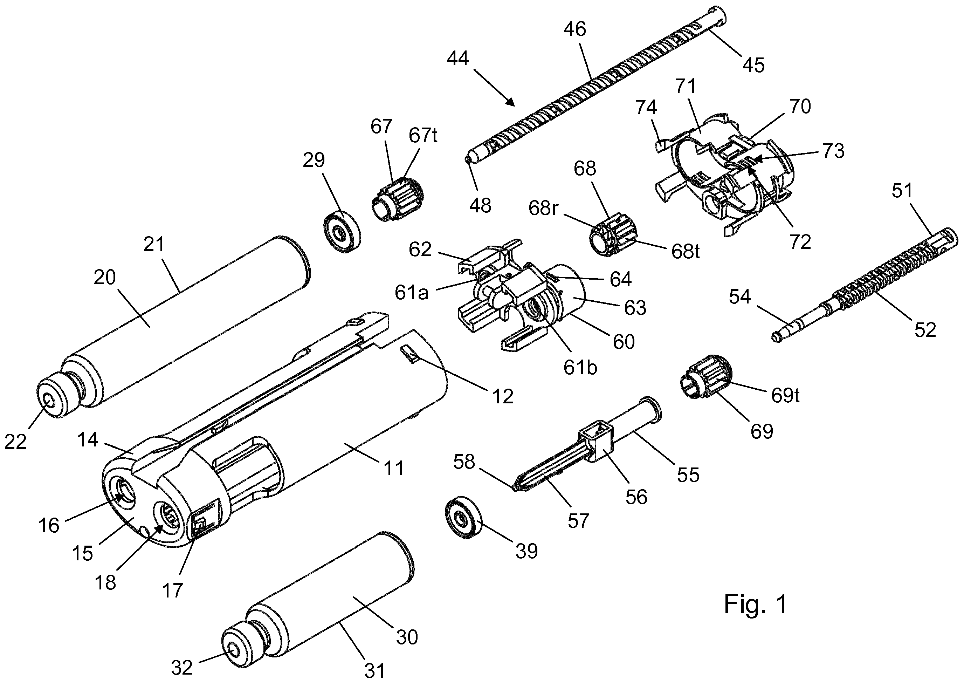

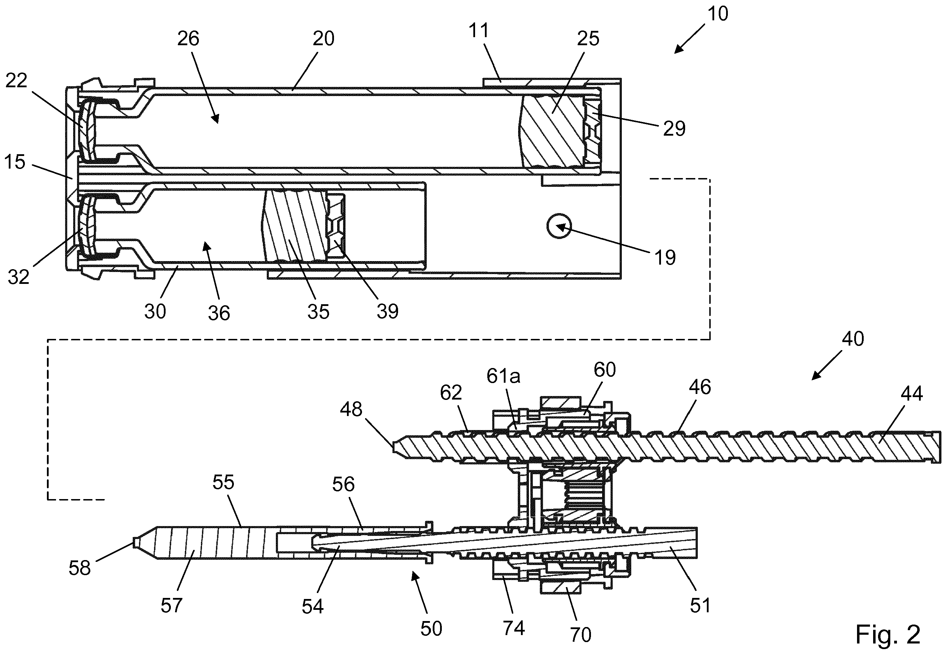

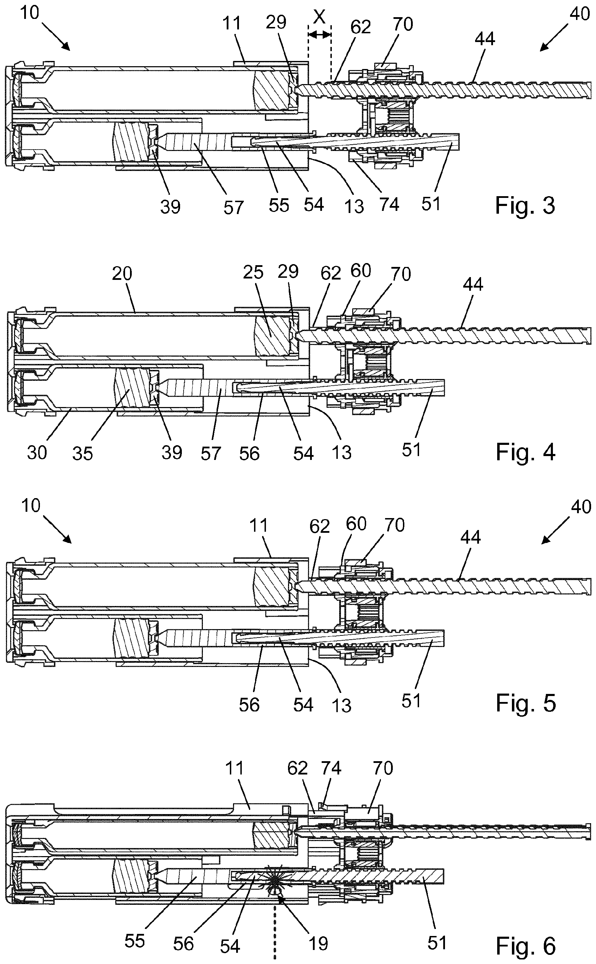

Fig. 1 is an exploded view of a reservoir sub-assembly 10 (Fig. 2) and a piston rod sub-assembly 40 (Fig. 2) of a drug delivery device 1 (Fig. 5) according to an embodiment of the invention. The reservoir sub-assembly 10 comprises a cartridge holder 11 of a generally oval shape, which enables accommodation of a first cartridge 20 holding a first medium and a second cartridge 30 holding a second medium. The first cartridge 20 has a generally cylindrical first cartridge body 21 and is closed by a first cartridge septum 22, respectively a first piston 25 (Fig. 2). The second cartridge 30 has a generally cylindrical second cartridge body 31 and is closed by a second cartridge septum 32, respectively a second piston 35 (Fig. 2). In this exemplary embodiment of the invention the second cartridge 30 is shorter than the first cartridge 20, and the first piston 25 is placed closer to the proximal end of the cartridge holder 11 than the second piston 35 is, the latter being clear e.g. from Fig. 2.

The cartridge holder 11 has a distal end portion 14 with a transversal distal end face 15, in which a first opening 16 and a second opening 18 are formed, and a side wall protrusion 17 for reception of a manifold needle unit (not shown). The first cartridge 20 and the second cartridge 30 are arranged in parallel within the cartridge holder 11 such that the first cartridge septum 22 is positioned adjacent the first opening 16 and the second cartridge septum 32 is positioned adjacent the second opening 18 to thereby allow for easy penetration of the respective cartridge septa by dedicated rear needles (not shown) in the manifold needle unit. At its proximal end portion the cartridge holder 11 is provided with a number of protrusions 12 for snap-fit engagement with a housing 2 (Fig. 5) of the drug delivery device 1. A first piston washer 29 is arranged in abutment with the first piston 25, and a second piston washer 39 is arranged in abutment with the second piston 35.

The piston rod sub-assembly 40 comprises a first piston rod structure 44, a second piston rod structure 50 (Fig. 2), a nut member 60, and a transmission case 70 having a bearing 71 which supports a first gearwheel 67, a second gearwheel 69, and a central gearwheel 68.

The first piston rod structure 44 comprises a first piston rod 45 which is provided with a non-self-locking thread 46 and has a first contact surface 48 adapted to abut the first piston washer 29 and apply a driving force to the first piston 25 therethrough.

The second piston rod structure 50 is a two-part structure. One part comprises a second piston rod 51 having a section with a self-locking thread 52 and a projecting portion 54. Another part comprises an extension 55 having a hollow rear portion 56 adapted to slidably receive the projecting portion 54 and a solid front portion 57 ending in a second contact surface 58 which is adapted to abut the second piston washer 39 and apply a driving force to the second piston 35 therethrough. In a pre-assembled state, before final assembly of the drug delivery device 1, the second piston rod 51 and the extension 55 are capable of sliding relative motion along a longitudinal axis of the second piston rod 51, and the axial extension of the second piston rod structure 50 is thereby variable.

In the present embodiment the first piston washer 29 and the second piston washer 39 form part of the reservoir sub-assembly 10. It is noted, however, that they may alternatively form part of the piston rod sub-assembly, the first piston washer 29 being attached to, or integral with, the first piston rod 45 at the first contact surface 48, and the second piston washer 39 being attached to, or integral with, the extension 55 at the second contact surface 58.

The nut member 60 comprises a first nut 61a, which is in threaded engagement with the non-self-locking thread 46 and thereby supports the first piston rod 45, and a second nut 61 b which is in threaded engagement with the self-locking thread 52 and thereby supports the second piston rod 51. Respective nut walls 63 extend proximally from the first nut 61a and the second nut 61b. Each nut wall 63 carries a protrusion 64 for coupling the nut member 60 to the transmission case 70, in a manner that will become clear from the below. The nut member 60 also has four legs 62 extending distally from the first nut 61a and the second nut 61b, arranged to interface with a proximal rim portion of the cartridge holder 11 during assembly of the drug delivery device 1.

In the bearing 71 the first gearwheel 67 has a first gearwheel toothing 67t, the second gearwheel 69 has a second gearwheel toothing 69t and the central gearwheel 68 has a central gearwheel toothing 68t which mesh with both the first gearwheel toothing 67t and the second gearwheel toothing 69t, thereby rotationally coupling the first gearwheel 67 and the second gearwheel 69. The central gearwheel 68 further has a ratchet toothing 68r arranged distally of the central gearwheel toothing 68t.

The transmission case 70 further comprises four guide rods 74 and a pair of unlocked state slots 72, respectively locked state slots 73 adapted for reception of the respective protrusions 64 on the nut walls 63 to define two distinct coupling states of the nut member 60 and the transmission case 70.

The first gearwheel 67 is arranged about and rotationally locked with respect to the first piston rod 45, and the second gearwheel 69 is arranged about and rotationally locked with respect to the second piston rod 51. A rotation of the first gearwheel 67 thus causes a corresponding rotation of the first piston rod 45, and vice versa, and a rotation of the second gearwheel 69 causes a corresponding rotation of the second piston rod 51, and vice versa.

Fig. 2 is a longitudinal section view of the reservoir sub-assembly 10 and the piston rod sub-assembly 40 in respective assembled states. The figure shows the parallel arrangement of the first cartridge 20, with a first chamber 26 holding first contents, and the second cartridge 30, with a second chamber 36 holding second contents, and reveals a bore 19 in the cartridge holder 11, the purpose of which will be described in the below.

The first piston rod 45 extends through the first nut 61a past the legs 62, and the first contact surface 48 is deliberately positioned more distally of the nut member 60 than it will be in the final assembly position of the first piston rod structure 44. Similarly, the second piston rod 51 extends through the second nut 61b, and the second contact surface 58 is deliberately positioned more distally of the nut member 60 than it will be in the final assembly position of the second piston rod structure 50. A portion of the projecting portion 54 resides within the hollow rear portion 56 of the extension 55 and is friction fitted therewith.

To ensure precision of the dose administration system it is essential that there is no air gap between the first piston washer 29 and the first piston rod structure 44, respectively between the second piston washer 39 and the second piston rod structure 50 in the final assembly state of the drug delivery device 1. A specific axial displacement of either piston rod structure as a consequence of an activation of a dose expelling mechanism (illustrated in Fig. 5) in the drug delivery device 1 is thereby transferred directly to the associated piston.

Accordingly, to eliminate the risk of potential air gaps a zero-point adjustment is performed by the manufacturer during assembly of the drug delivery device 1. The construction of the piston rod sub-assembly 40 allows for a particularly simple zero-point adjustment procedure, as will be described hereafter with reference to Figs. 3-6.

Fig. 3 is a longitudinal section view of the reservoir sub-assembly 10 and the piston rod sub-assembly 40 at the onset of the zero-point adjustment procedure where the first piston washer 29 has been brought in abutment with the first contact surface 48, by converging relative axial motion between the cartridge holder 11 and the transmission case 70, and there is a clearance, X, between the proximal rim of the cartridge holder 11 and the distal end of the respective legs 62. The transmission case 70 is axially and rotationally fixed in a housing 2 (Fig. 5) of the drug delivery device 1 at this stage. However, for the sake of clarity the housing 2 has been omitted from Figs. 3-6.

The initial respective positions of the first contact surface 48 and the second contact surface 58 relative to the nut member 60 are in principle correlated with the respective positions of the first piston 25 in the first cartridge body 21 and the second piston 35 in the second cartridge body 31, and Fig. 3 does in fact also show that the second piston washer 39 abuts the second contact surface 58. However, due to various manufacturing tolerances the second piston washer 39 may not be brought in abutment with the second contact surface 58 at the same time as the first piston washer 29 touches the first contact surface 48. Regardless of which contact is established first though, the design of the piston rod sub-assembly 40 ensures that the second contact is established subsequently by mere converging relative axial motion between the cartridge holder 11 and the transmission case 70.

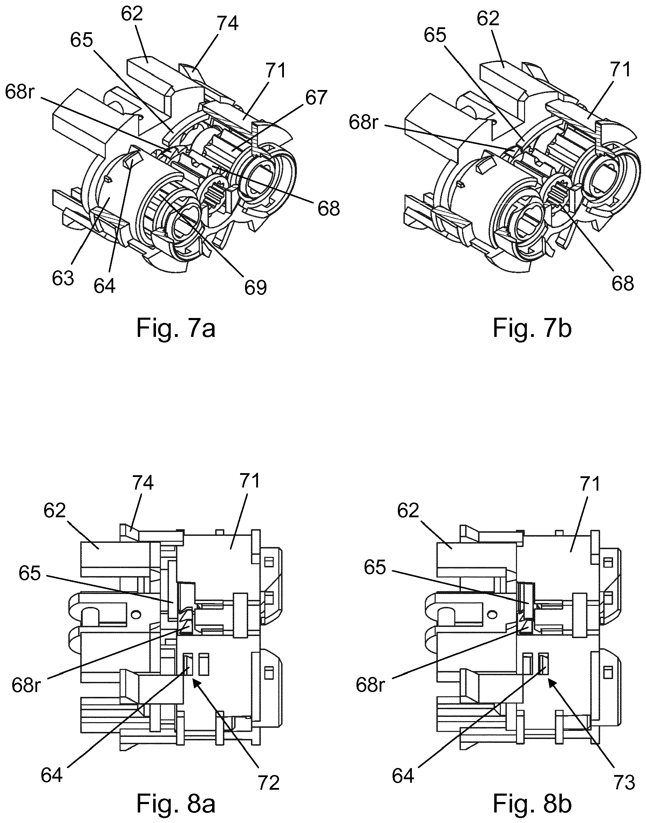

In the shown state of the piston rod sub-assembly 40, which is further illustrated in Figs. 7a and 8a showing a perspective view, respectively a side view of the nut member 60 and the transmission case 70 in a first of the aforementioned two distinct coupling states (the transmission case being partially sectioned in the perspective view for the sake of clarity), the central gearwheel 68 is capable of rotation in both directions about its own axis of extension. This is because the protrusion 64 occupies the unlocked state slot 72 (Fig. 8a) and the ratchet arm 65 is axially spaced apart from the ratchet toothing 68r.

Hence, if the first piston washer 29 reaches the first contact surface 48 before the second piston washer 39 reaches the second contact surface 58 a continued converging relative axial motion between the cartridge holder 11 and the transmission case 70 will cause the piston washer 29 to press the first piston rod 45 backwards due to the first chamber 26 being filled with an incompressible liquid. Since the first piston rod 45 is engaged with the first nut 61a via the non-self-locking thread 46 the axial force from the first piston washer 29 will cause a helical proximal motion of the first piston rod 45 as it rotates through the first nut 61a.

The rotation of the first piston rod 45 will cause a rotation of the first gearwheel 67, which, due to the meshing of the first gearwheel toothing 67t and the central gearwheel toothing 68t, will cause a rotation of the central gearwheel 68, which in turn, due to the meshing of the central gearwheel toothing 68t and the second gearwheel toothing 69t, will cause a rotation of the second gearwheel 69 and thereby a helical proximal motion of the second piston rod 51. Notably, the axial displacement of the second piston rod 51 will be smaller than that of the first piston rod 45 because the self-locking thread 52 has a smaller pitch than the non-self-locking thread 46. Hence, eventually, during the pressing back of the first piston rod 45 the second piston washer 39 will be brought in abutment with the second contact surface 58.

Alternatively, if the second piston washer 39 reaches the second contact surface 58 before the first piston washer 29 reaches the first contact surface 48 a continued converging relative axial motion between the cartridge holder 11 and the transmission case 70 will simply cause the extension 55 to be pushed axially backwards over the projecting portion 54 until the first piston washer 29 is brought in abutment with the first contact surface 48.

After establishment of the initial contact between the first piston washer 29 and the first piston rod 45, shown in Fig. 3, the cartridge holder 11 and the transmission case 70 undergo further converging relative axial motion, eliminating the clearance, X. In Fig. 4 the proximal rim of the cartridge holder 11 has thereby been brought in abutment with the respective legs 62, and at this point the first piston washer 29 abuts the first piston rod 45, while the second piston washer 39 abuts the extension 55. In other words, both parts of the dose administration system have been zero-point adjusted.

During the converging relative axial motion between the cartridge holder 11 and the transmission case 70 which eliminates the clearance, X, the first piston rod structure 44 undergoes helical proximal motion due to the non-self-locking thread 46 of the first piston rod 45, whereas the second piston rod structure 50 undergoes a combined helical proximal motion of the second piston rod 51 and a pure axial proximal motion of the extension 55, the former driven by the intermeshing gearwheels in the transmission case 70 and the latter by the axial force from the second piston washer 39. Since the axial component of the helical proximal motion of the second piston rod 51 is smaller than the axial proximal motion of the extension 55 the second piston rod structure 50 changes axial dimension until the cartridge holder 11 and the respective legs 62 interact, at which point the projecting portion 54 has reached its final position in the hollow rear portion 56.

Further converging relative axial motion between the cartridge holder 11 and the transmission case 70 after contact is established between the proximal rim of the cartridge holder 11 and the legs 62 will force the nut member 60 towards the transmission case 70 and cause the protrusion 64 to snap out of the unlocked state slot 72 and into the locked state slot 73, thereby interlocking the nut member 60 and the transmission case 70 in a second of the aforementioned two distinct coupling states. In this second coupling state, which is illustrated by a perspective, partially sectioned, view in Fig. 7b and a side view in Fig. 8b, the ratchet arm 65 is axially aligned, and in engagement, with the ratchet toothing 68r, ensuring unidirectional rotational motion of the central gearwheel 68.

The central gearwheel 68 is thereby allowed to rotate in a direction which causes a direction of rotation of the first gearwheel 67 and the second gearwheel 69 that corresponds to respective advancements of the first piston rod 45 through the first nut 61a and the second piston rod 51 through the second nut 61b, but not allowed to rotate in the opposite direction. The first gearwheel 67 and the second gearwheel 69 are consequently also restricted to unidirectional rotational motion. This means that the first piston rod 45 and the second piston rod 51 can no longer undergo proximal displacement relative to the nut member 60, and the established contact between the first contact surface 48 and the first piston washer 29, respectively the second contact surface 58 and the second piston washer 39, is accordingly secured.

Notably, the joint motion of the cartridge holder 11 and the nut member 60 relative to the transmission case 70 does not cause any rotation of the first piston rod structure 44 and the second piston rod structure 50, since the first piston rod 45 and the second piston rod 51 are shifted proximally together with the first nut 61a and the second nut 61b. Furthermore, since the second piston rod 51 is shifted the same distance as the second piston washer 39 the second piston rod structure 50 does not undergo a further reduction in axial dimension. The resulting interrelated positions of the various components of the reservoir sub-assembly 10 and the piston rod sub-assembly 40, constituting the final assembly state of these subassemblies, are depicted in Fig. 5.

Fig. 6 is a v-section view of the reservoir sub-assembly 10 and the piston rod sub-assembly 40 in their final assembly state, showing that the extension 55 after having assumed the above described position relative to the second piston rod 51 is subsequently fixed thereto by laser welding a wall portion of the hollow rear portion 56 to the projecting portion 54 through the bore 19. Thereby, a second piston rod structure 50 with a non-adjustable axial dimension, which is capable of transferring an axial force to the second piston washer 39 to drive the second piston 35, is provided.

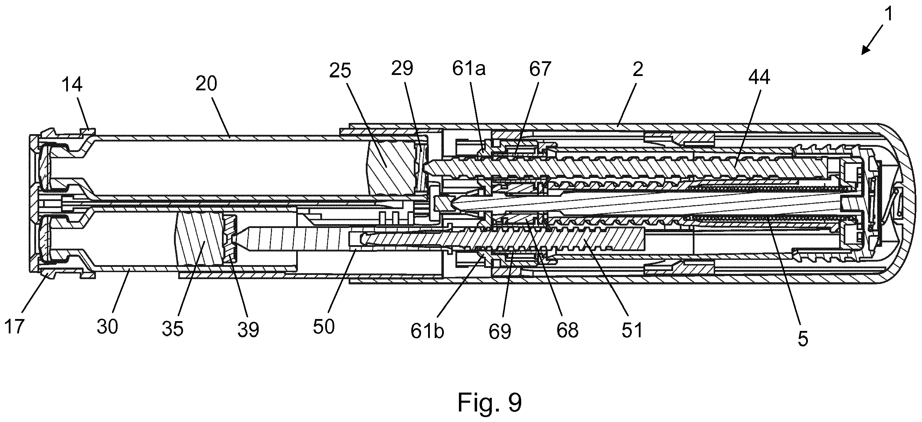

Fig. 9 is a longitudinal section view of the assembled drug delivery device 1, where the cartridge holder 11 is connected to the housing 2. Due to the above described zero-point adjustment the first piston rod structure 44 is in contact with the first piston washer 29 which is in contact with the first piston 25 in the first cartridge 20, and the, now rigid, second piston rod structure 50 is in contact with the second piston washer 39 which is in contact with the second piston 35 in the second cartridge 30. The dosing system is thus pre-primed and an activation of the two piston rod structures via the dose expelling mechanism will accordingly lead to an immediate axial displacement of the two pistons. The dose expelling mechanism is powered by a tensioned torsion spring 5 which is releasable to cause a rotation of the central gearwheel 68 in the transmission case 70. Details of the release mechanism for the torsion spring 5 as well as further details of the drive mechanism for the piston rod structures are not provided herein, since these are irrelevant to the present invention.

It is noted that whereas the described exemplary embodiment of the invention concerns a twin chamber type of drug delivery device the overarching principle of zero-point adjustment disclosed in the above applies equally to a drug delivery device with a single drug reservoir, such as a fountain pen shaped injection device. In that case the cartridge holder 11 is adapted to carry a single cartridge with a piston, the housing 2 accommodates a single piston rod (having a non-self-locking thread) and, instead of rotationally coupled gearwheels, a single piston rod drive member, e.g. itself carrying a ratchet toothing, and the nut member 60 comprises a single nut and a ratchet arm for engagement with the ratchet toothing after contact between the piston and the piston rod is established. The mechanism for rotating the piston rod drive member during a dose expelling event may in principle be any of a number of known drive mechanisms, a specific example being the one sketched in WO 2017/072233. The nut member may be retained in the first nut member position and/or the second nut member position by respective snap fittings or friction fittings with e.g. the housing.

## Claims

1. A drug delivery device (1) comprising:
- a cartridge unit (10) comprising a cartridge holder (11) carrying a drug cartridge (20) having a cartridge body (21) and a piston structure (25, 29), and
- a dose expelling unit comprising
∘ a housing (2) extending along a longitudinal axis,
∘ a piston rod (45) for pressurising the drug cartridge (20), the piston rod (45) comprising a contact surface (48) adapted to contact the piston structure (25, 29), and a non-self-locking thread (46),
∘ a nut member (60) rotationally fixed with respect to the housing (2) and engaged with the non-self-locking thread (46), and
∘ a piston rod drive member (67) axially fixed with respect to the housing (2) and rotatable about the longitudinal axis,
wherein the piston rod drive member (67) and the piston rod (45) are rotationally interlocked, such that a rotation of the piston rod drive member (67) in a first direction is associated with distal movement of the piston rod (45) through the nut member (60), and a rotation of the piston rod drive member (67) in a second direction is associated with proximal movement of the piston rod (45) through the nut member (60),
**characterised in that** the nut member (60) carries a first coupling part (65) of a unidirectional ratchet coupling (65, 68r), and the piston rod drive member (67) is operatively coupled with a second coupling part (68r) of the unidirectional ratchet coupling (65, 68r),
wherein in a pre-assembled state the nut member (60) assumes a first nut member position relative to the housing (2) and the piston rod drive member (67) in which the first coupling part (65) and the second coupling part (68r) are disengaged and in an assembled state the nut member (60) assumes a second nut member position relative to the housing (2) and the piston rod drive member (67) in which the first coupling part (65) and the second coupling part (68r) are engaged to render the piston rod drive member (67) unidirectionally rotatable in the first direction, and
wherein the nut member (60) is configured to undergo irreversible displacement from the first nut member position to the second nut member position during assembly of the dose expelling unit and the cartridge unit (10) in response to a converging relative axial motion between the housing (2) and the cartridge holder (11), after contact between the piston structure (25, 29) and the contact surface (48) has been established.

2. A drug delivery device according to claim 1, wherein the cartridge holder (11) comprises a proximal rim portion (13) which applies a proximally directed force to the nut member (60) during the converging relative axial motion between the housing (2) and the cartridge holder (11), the proximally directed force bringing the nut member (60) from the first nut member position to the second nut member position.

3. A drug delivery device according to claim 1 or 2, wherein the cartridge unit (10) further comprises
- a second drug cartridge (30) having a second cartridge body (31) and a second piston structure (35, 39), the drug cartridge (20) and the second drug cartridge (30) being held in parallel by the cartridge holder (11),
and the dose expelling unit further comprises
- a second piston rod (50) for pressurising the second drug cartridge (30), the second piston rod (50) comprising a second contact surface (58) adapted to contact the second piston structure (35, 39), and a second piston rod thread (52) in engagement with a second thread of the nut member (60), and
- a second piston rod drive member (69) axially fixed with respect to the housing (2) and rotatable about a second axis parallel with the longitudinal axis, the second piston rod drive member (69) and the second piston rod (50) being rotationally interlocked, and the second piston rod drive member (69) being rotationally coupled with the piston rod drive member (67), and
wherein the second piston rod (50) has an axially variable dimension which is set during assembly of the dose expelling unit and the cartridge unit (10), after contact between the second piston structure (35, 39) and the second piston rod (50) has been established.

4. A drug delivery device according to claim 3, wherein the second piston rod (50) comprises a front piston rod portion (51) and a rear piston rod portion (55) capable of undergoing relative sliding motion along one another, and wherein the axially variable dimension is set by axially interlocking the front piston rod portion (51) and the rear piston rod portion (55).

5. A drug delivery device according to claim 4, wherein the piston rod drive member (67) and the second piston rod drive member (69) are arranged in a casing (70) which is fixed to the housing (2), wherein the casing (70) is provided with a distal slot (72) and a proximal slot (73) axially spaced apart from the distal slot (72), and wherein the nut member (60) comprises a protrusion (64) adapted to move from a position in the distal slot (72) to a position in the proximal slot (73) during movement of the nut member (60) from the first nut member position to the second nut member position and structured to prevent subsequent movement of the nut member (60) towards the first nut member position.

6. A drug delivery device according to claim 4 or 5, further comprising a central gearwheel (68), wherein the piston rod drive member (67) comprises a first gearwheel toothing (67t) and the second piston rod drive member (69) comprises a second gearwheel toothing (69t), and wherein the piston rod drive member (67) and the second piston rod drive member (69) are rotationally coupled via the central gearwheel (68).

7. A drug delivery device according to claim 6, wherein the second coupling part (68r) forms part of the central gearwheel (68).

8. A drug delivery device according to any of claims 4-7, wherein the second piston rod thread (52) is self-locking.

9. A drug delivery device according to any of claims 4-8, wherein the front piston rod portion (51) and the rear piston rod portion (55) are interlocked by laser welding through a bore (19) in the cartridge holder (11).

10. A drug delivery device according to any of claims 4-9, wherein the drug cartridge (20) further has a drug outlet sealed by a penetrable septum (22), and the second drug cartridge (30) further has a second drug outlet sealed by a second penetrable septum (32), and wherein when the penetrable septum (22) and the second penetrable septum (32) are axially aligned relative to the cartridge holder (11) the second piston structure (35, 39) is positioned distally of the piston structure (25, 29).

11. A method of performing zero-point adjustment of a drug delivery device (1) comprising A) a cartridge unit (10) comprising a cartridge holder (11) carrying a drug cartridge (20) having a cartridge body (21) and a piston structure (25, 29), and B) a dose expelling unit comprising a housing (2) extending along a longitudinal axis, a piston rod (45) for pressurising the drug cartridge (20), the piston rod (45) comprising a contact surface (48) adapted to contact the piston structure (25, 29), and a non-self-locking thread (46), a nut member (60) rotationally fixed with respect to the housing (2) and engaged with the non-self-locking thread (46), the nut member (60) carrying a first coupling part (65) of a unidirectional ratchet coupling (65, 68r), and a piston rod drive member (67) axially fixed with respect to the housing (2) and rotatable about the longitudinal axis, the piston rod drive member (67) being operatively coupled with a second coupling part (68r) of the unidirectional ratchet coupling (65, 68r), wherein the piston rod drive member (67) and the piston rod (45) are rotationally interlocked, such that a rotation of the piston rod drive member (67) in a first direction is associated with distal helical movement of the piston rod (45) in the nut member (60), and a rotation of the piston rod drive member (67) in a second direction is associated with proximal helical movement of the piston rod (45) in the nut member (60), the method comprising:
(i) arranging the piston rod (45) in the nut member (60) such that the contact surface (48) is positioned distally of a final contact surface assembly position,
(ii) bringing the piston structure (25, 29) and the contact surface (48) into mutual abutment,
(iii) inducing a converging relative axial motion between the cartridge holder (11) and the housing (2), thereby firstly forcing the piston rod (45) proximally in the nut member (60) by means of the piston structure (25, 29) and secondly moving the nut member (60) axially relative to the housing (2) from a first nut member position in which the first coupling part (65) and the second coupling part (68r) are disengaged to a second nut member position in which the first coupling part (65) and the second coupling part (68r) are engaged, rendering the piston rod drive member (67) unidirectionally rotatable in the first direction, and
(iv) axially interlocking the cartridge holder (11) and the housing (2).

12. A method according to claim 11, wherein bringing the piston structure (25, 29) and the contact surface (48) into mutual abutment involves inducing a converging relative axial motion between the cartridge holder (11) and the housing (2).

13. A method of performing zero-point adjustment of a drug delivery device (1) comprising
- a cartridge unit (10) comprising a cartridge holder (11) carrying
∘ a first drug cartridge (20) having a first cartridge body (21) and a first piston structure (25, 29), and
∘ a second drug cartridge (30) having a second cartridge body (31) and a second piston structure (35, 39), and
- a dose expelling unit comprising
∘ a housing (2) extending along a longitudinal central axis,
∘ a first piston rod (45) for pressurising the first drug cartridge (20), the first piston rod (45) comprising a first contact surface (48) adapted to contact the first piston structure (25, 29), and a non-self-locking thread (46),
∘ a second piston rod (50) for pressurising the second drug cartridge (30), the second piston rod (50) comprising a front piston rod portion (51) having a second contact surface (58) adapted to contact the second piston structure (35, 39), and a rear rod portion (55) having a second piston rod thread (52), the front piston rod portion (51) and the rear piston rod portion (55) being capable of undergoing relative sliding motion along one another,
∘ a nut member (60) rotationally fixed with respect to the housing (2) and respectively engaged with the non-self-locking thread (46) and the second piston rod thread (52), the nut member (60) carrying a first coupling part (65) of a unidirectional ratchet coupling (65, 68r),
∘ a first piston rod drive member (67) axially fixed with respect to the housing (2) and rotatable about a first axis parallel with the longitudinal central axis, the first piston rod drive member (67) being operatively coupled with a second coupling part (68r) of the unidirectional ratchet coupling (65, 68r), and the first piston rod drive member (67) and the first piston rod (45) being rotationally interlocked such that a rotation of the first piston rod drive member (67) in a first direction about the first axis is associated with distal helical movement of the first piston rod (45) in the nut member (60), and a rotation of the first piston rod drive member (67) in a second direction about the first axis is associated with proximal helical movement of the first piston rod (45) in the nut member (60), and
∘ a second piston rod drive member (69) axially fixed with respect to the housing (2) and rotatable about a second axis parallel with the longitudinal central axis, the second piston rod drive member (69) being rotationally coupled with the first piston rod drive member (67), and the second piston rod drive member (69) and the rear piston rod portion (55) being rotationally interlocked such that a rotation of the second piston rod drive member (69) in a first direction about the second axis is associated with distal helical movement of the rear piston rod portion (55) in the nut member (60), and a rotation of the second piston rod drive member (69) in a second direction about the second axis is associated with proximal helical movement of the rear piston rod portion (55) in the nut member (60),
the method comprising:
(i) arranging the first piston rod (45) and the second piston rod (50) in the nut member (60) such that the first contact surface (48) is positioned distally of a final first contact surface assembly position and the second contact surface (58) is positioned distally of a final second contact surface assembly position,
(ii) aligning the cartridge holder (11) and the housing (2) along the longitudinal central axis such that the first piston structure (25, 29) is aligned with the first axis and the second piston structure (35, 39) is aligned with the second axis,
(iii) inducing a converging relative axial motion between the cartridge holder (11) and the housing (2), said motion comprising a first part motion which brings the cartridge holder (11) and the housing (2) to an intermediate assembly position and in the course of which the first contact surface (48) is moved proximally relative to the nut member (60) by means of the first piston structure (25, 29) and the second contact surface (58) is moved proximally relative to the rear piston rod portion (55), the second piston rod (50) thereby obtaining an intermediate second piston rod assembly configuration, and a second part motion which brings the cartridge holder (11) and the housing (2) to a final assembly position and during which the first piston rod (45), the second piston rod (50), and the nut member (60) are moved jointly relative to the first piston rod drive member (67), the first coupling part (65) of the unidirectional ratchet coupling (65, 68r) thereby entering into engagement with the second coupling part (68r) of the unidirectional ratchet coupling (65, 68r), rendering the first piston rod drive member (67) unidirectionally rotatable in the first direction about the first axis and the second piston rod drive member (67) unidirectionally rotatable in the first direction about the second axis,
(iv) axially interlocking the front piston rod portion (51) and the rear piston rod portion (55) in the intermediate second piston rod assembly configuration, and
(v) axially interlocking the cartridge holder (11) and the housing (2) in the final assembly position.

## Patentansprüche

1. Arzneimittelabgabevorrichtung (1), umfassend:
- eine Kartuscheneinheit (10), die einen Kartuschenhalter (11) umfasst, der eine Arzneimittelkartusche (20) mit einem Kartuschenkörper (21) und einer Kolbenstruktur (25, 29) trägt, und
- eine Dosisausstoßeinheit, umfassend
∘ ein Gehäuse (2), das sich entlang einer Längsachse erstreckt,
∘ eine Kolbenstange (45) zur Druckbeaufschlagung der Arzneimittelkartusche (20), wobei die Kolbenstange (45) eine Kontaktfläche (48), die dazu ausgelegt ist, die Kolbenstruktur (25, 29) zu berühren, und ein nicht selbstsicherndes Gewinde (46) umfasst,
∘ ein Mutterelement (60), das in Bezug auf das Gehäuse (2) drehfest ist und mit dem nicht selbstsichernden Gewinde (46) in Eingriff steht, und
∘ ein Kolbenstangen-Antriebselement (67), das in Bezug auf das Gehäuse (2) axial fixiert und um die Längsachse drehbar ist,
wobei das Kolbenstangen-Antriebselement (67) und die Kolbenstange (45) drehfest miteinander verbunden sind, sodass eine Drehung des Kolbenstangen-Antriebselements (67) in eine erste Richtung mit einer distalen Bewegung der Kolbenstange (45) durch das Mutterelement (60) verbunden ist und eine Drehung des Kolbenstangen-Antriebselements (67) in eine zweite Richtung mit einer proximalen Bewegung der Kolbenstange (45) durch das Mutterelement (60) verbunden ist,
**dadurch gekennzeichnet, dass** das Mutterelement (60) ein erstes Kupplungsteil (65) einer unidirektionalen Ratschenkupplung (65, 68r) trägt und das Kolbenstangen-Antriebselement (67) betriebsmäßig mit einem zweiten Kupplungsteil (68r) der unidirektionalen Ratschenkupplung (65, 68r) gekoppelt ist,
wobei das Mutterelement (60) in einem vormontierten Zustand eine erste Mutterelementposition relativ zu dem Gehäuse (2) und dem Kolbenstangen-Antriebselement (67) einnimmt, in der das erste Kupplungsteil (65) und das zweite Kupplungsteil (68r) außer Eingriff sind, wobei das Mutterelement (60) in einem montierten Zustand eine zweite Mutterelementposition relativ zu dem Gehäuse (2) und dem Kolbenstangen-Antriebselement (67) einnimmt, in der das erste Kupplungsteil (65) und das zweite Kupplungsteil (68r) in Eingriff stehen, um das Kolbenstangen-Antriebselement (67) unidirektional in die erste Richtung drehbar zu machen, und
wobei das Mutterelement (60) so konfiguriert ist, dass es während der Montage der Dosisausstoßeinheit und der Kartuscheneinheit (10) in Reaktion auf eine konvergierende relative axiale Bewegung zwischen dem Gehäuse (2) und dem Kartuschenhalter (11) eine irreversible Verschiebung von der ersten Mutterelementposition zu der zweiten Mutterelementposition erfährt, nachdem der Kontakt zwischen der Kolbenstruktur (25, 29) und der Kontaktfläche (48) hergestellt wurde.

2. Arzneimittelabgabevorrichtung nach Anspruch 1, wobei der Kartuschenhalter (11) einen proximalen Randabschnitt (13) aufweist, der während der konvergierenden relativen axialen Bewegung zwischen dem Gehäuse (2) und dem Kartuschenhalter (11) eine proximal gerichtete Kraft auf das Mutterelement (60) ausübt, wobei die proximal gerichtete Kraft das Mutterelement (60) von der ersten Mutterelementposition in die zweite Mutterelementposition überführt.

3. Arzneimittelabgabevorrichtung nach Anspruch 1 oder 2, wobei die Kartuscheneinheit (10) ferner umfasst:
- eine zweite Arzneimittelkartusche (30) mit einem zweiten Kartuschenkörper (31) und einer zweiten Kolbenstruktur (35, 39), wobei die Arzneimittelkartusche (20) und die zweite Arzneimittelkartusche (30) parallel durch den Kartuschenhalter (11) gehalten werden,
und die Dosisausstoßeinheit ferner umfasst:
- eine zweite Kolbenstange (50) zum Druckbeaufschlagen der zweiten Arzneimittelkartusche (30), wobei die zweite Kolbenstange (50) eine zweite Kontaktfläche (58), die dazu ausgelegt ist, die zweite Kolbenstruktur (35, 39) zu berühren, und ein zweites Kolbenstangengewinde (52) in Eingriff mit einem zweiten Gewinde des Mutterelements (60) umfasst, und
- ein zweites Kolbenstangen-Antriebselement (69), das in Bezug auf das Gehäuse (2) axial fixiert und um eine zweite Achse parallel zu der Längsachse drehbar ist, wobei das zweite Kolbenstangen-Antriebselement (69) und die zweite Kolbenstange (50) drehfest miteinander verbunden sind und das zweite Kolbenstangen-Antriebselement (69) mit dem Kolbenstangen-Antriebselement (67) drehgekoppelt ist, und
wobei die zweite Kolbenstange (50) eine axial variable Abmessung aufweist, die während der Montage der Dosisausstoßeinheit und der Kartuscheneinheit (10) eingestellt wird, nachdem der Kontakt zwischen der zweiten Kolbenstruktur (35, 39) und der zweiten Kolbenstange (50) hergestellt wurde.

4. Arzneimittelabgabevorrichtung nach Anspruch 3, wobei die zweite Kolbenstange (50) einen vorderen Kolbenstangenabschnitt (51) und einen hinteren Kolbenstangenabschnitt (55) aufweist, die eine relative Gleitbewegung aneinander entlang ausführen können, und wobei die axial variable Abmessung durch eine axiale Verbindung des vorderen Kolbenstangenabschnitts (51) und des hinteren Kolbenstangenabschnitts (55) eingestellt wird.

5. Arzneimittelabgabevorrichtung nach Anspruch 4, wobei das Kolbenstangen-Antriebselement (67) und das zweite Kolbenstangen-Antriebselement (69) in einem Gehäuse (70) angeordnet sind, das an dem Gehäuse (2) befestigt ist, wobei das Gehäuse (70) mit einem distalen Schlitz (72) und einem proximalen Schlitz (73) versehen ist, der axial von dem distalen Schlitz (72) beabstandet ist, und wobei das Mutterelement (60) einen Vorsprung (64) umfasst, der dazu ausgelegt ist, sich während der Bewegung des Mutterelements (60) von der ersten Mutterelementposition zu der zweiten Mutterelementposition von einer Position in dem distalen Schlitz (72) zu einer Position in dem proximalen Schlitz (73) zu bewegen, und so strukturiert ist, dass er eine nachfolgende Bewegung des Mutterelements (60) in Richtung der ersten Mutterelementposition verhindert.

6. Arzneimittelabgabevorrichtung nach Anspruch 4 oder 5, die ferner ein zentrales Zahnrad (68) aufweist, wobei das Kolbenstangen-Antriebselement (67) eine erste Zahnradverzahnung (67t) aufweist und das zweite Kolbenstangen-Antriebselement (69) eine zweite Zahnradverzahnung (69t) aufweist, und wobei das Kolbenstangen-Antriebselement (67) und das zweite Kolbenstangen-Antriebselement (69) über das zentrale Zahnrad (68) drehgekoppelt sind.

7. Arzneimittelabgabevorrichtung nach Anspruch 6, wobei das zweite Kupplungsteil (68r) einen Teil des zentralen Zahnrads (68) bildet.

8. Arzneimittelabgabevorrichtung nach einem der Ansprüche 4-7, wobei das zweite Kolbenstangengewinde (52) selbstsichernd ist.

9. Arzneimittelabgabevorrichtung nach einem der Ansprüche 4-8, wobei der vordere Kolbenstangenabschnitt (51) und der hintere Kolbenstangenabschnitt (55) durch Laserschweißen durch eine Bohrung (19) in dem Kartuschenhalter (11) miteinander verbunden sind.

10. Arzneimittelabgabevorrichtung nach einem der Ansprüche 4-9, wobei die Arzneimittelkartusche (20) ferner einen Arzneimittelauslass aufweist, der durch eine durchdringbare Membran (22) verschlossen ist, und die zweite Arzneimittelkartusche (30) ferner einen zweiten Arzneimittelauslass aufweist, der durch eine zweite durchdringbare Membran (32) verschlossen ist, wobei die zweite Kolbenstruktur (35, 39) distal von der Kolbenstruktur (25, 29) positioniert ist, wenn die durchdringbare Membran (22) und die zweite durchdringbare Membran (32) axial relativ zu dem Kartuschenhalter (11) ausgerichtet sind.

11. Verfahren zum Durchführen einer Nullpunkteinstellung einer Arzneimittelabgabevorrichtung (1), umfassend A) eine Kartuscheneinheit (10), die einen Kartuschenhalter (11) umfasst, der eine Arzneimittelkartusche (20) trägt, die einen Kartuschenkörper (21) und eine Kolbenstruktur (25, 29) aufweist, und B) eine Dosisausstoßeinheit, die ein Gehäuse (2), das sich entlang einer Längsachse erstreckt, eine Kolbenstange (45) zum Druckbeaufschlagen der Arzneimittelkartusche (20), wobei die Kolbenstange (45) eine Kontaktfläche (48), die dazu ausgelegt ist, die Kolbenstruktur (25, 29) zu berühren, und ein nicht selbstsicherndes Gewinde (46) umfasst, ein Mutterelement (60), das in Bezug auf das Gehäuse (2) drehfest ist und mit dem nicht selbstsichernden Gewinde (46) in Eingriff steht, wobei Mutterelement (60) ein erstes Kupplungsteil (65) einer unidirektionalen Ratschenkupplung (65, 68r) trägt, und ein Kolbenstangen-Antriebselement (67), das in Bezug auf das Gehäuse (2) axial fixiert und um die Längsachse drehbar ist, wobei das Kolbenstangen-Antriebselement (67) mit einem zweiten Kupplungsteil (68r) der unidirektionalen Ratschenkupplung (65, 68r) betriebsmäßig gekoppelt ist, wobei das Kolbenstangen-Antriebselement (67) und die Kolbenstange (45) drehfest miteinander verbunden sind, sodass eine Drehung des Kolbenstangen-Antriebselements (67) in eine erste Richtung mit einer distalen schraubenförmigen Bewegung der Kolbenstange (45) in dem Mutterelement (60) verbunden ist, und eine Drehung des Kolbenstangen-Antriebselements (67) in eine zweite Richtung mit einer proximalen schraubenförmigen Bewegung der Kolbenstange (45) in dem Mutterelement (60) verbunden ist, umfasst, wobei das Verfahren umfasst:
(i) Anordnen der Kolbenstange (45) in dem Mutterelement (60), sodass die Kontaktfläche (48) distal von einer abschließenden Kontaktflächenmontageposition positioniert ist,
(ii) Inanlagebringen der Kolbenstruktur (25, 29) und der Kontaktfläche (48),
(iii) Herbeiführen einer konvergierenden relativen axialen Bewegung zwischen dem Kartuschenhalter (11) und dem Gehäuse (2), wodurch zunächst die Kolbenstange (45) mithilfe der Kolbenstruktur (25, 29) proximal in das Mutterelement (60) gezwungen wird und des Weiteren das Mutterelement (60) axial relativ zu dem Gehäuse (2) von einer ersten Mutterelementposition, in der das erste Kupplungsteil (65) und das zweite Kupplungsteil (68r) außer Eingriff sind, in eine zweite Mutterelementposition bewegt wird, in der das erste Kupplungsteil (65) und das zweite Kupplungsteil (68r) in Eingriff stehen, wodurch das Kolbenstangen-Antriebselement (67) unidirektional in die erste Richtung drehbar wird, und
(iv) axiales Verbinden des Kartuschenhalters (11) und des Gehäuses (2).

12. Verfahren nach Anspruch 11, wobei das Inanlagebringen der Kolbenstruktur (25, 29) und der Kontaktfläche (48) das Herbeiführen einer konvergierenden relativen axialen Bewegung zwischen dem Kartuschenhalter (11) und dem Gehäuse (2) beinhaltet.

13. Verfahren zur Durchführung einer Nullpunkteinstellung einer Arzneimittelabgabevorrichtung (1), umfassend
- eine Kartuscheneinheit (10), die einen Kartuschenhalter (11) umfasst, der Folgendes trägt:
∘ eine erste Arzneimittelkartusche (20), die einen ersten Kartuschenkörper (21) und eine ersten Kolbenstruktur (25, 29) aufweist, und
∘ eine zweite Arzneimittelkartusche (30), die einen zweiten Kartuschenkörper (31) und eine zweite Kolbenstruktur (35, 39) aufweist, und
- eine Dosisausstoßeinheit, umfassend
∘ ein Gehäuse (2), das sich entlang einer zentralen Längsachse erstreckt,
∘ eine erste Kolbenstange (45) zum Druckbeaufschlagen der ersten Arzneimittelkartusche (20), wobei die erste Kolbenstange (45) eine erste Kontaktfläche (48), die dazu ausgelegt ist, die erste Kolbenstruktur (25, 29) zu berühren, und ein nicht selbssicherndes Gewinde (46) umfasst,
o eine zweite Kolbenstange (50) zum Druckbeaufschlagen der zweiten Arzneimittelkartusche (30), wobei die zweite Kolbenstange (50) einen vorderen Kolbenstangenabschnitt (51) mit einer zweiten Kontaktfläche (58), die dazu ausgelegt ist, die zweite Kolbenstruktur (35, 39) zu berühren, und einen hinteren Kolbenstangenabschnitt (55) mit einem zweiten Kolbenstangengewinde (52) umfasst, wobei der vordere Kolbenstangenabschnitt (51) und der hintere Kolbenstangenabschnitt (55) eine relative Gleitbewegung aneinander entlang ausführen können,
∘ ein Mutterelement (60), das in Bezug auf das Gehäuse (2) drehfest ist und jeweils mit dem nicht selbstsichernden Gewinde (46) und dem zweiten Kolbenstangengewinde (52) in Eingriff steht, wobei das Mutterelement (60) ein erstes Kupplungsteil (65) einer unidirektionalen Ratschenkupplung (65, 68r) trägt,
o ein erstes Kolbenstangen-Antriebselement (67), das in Bezug auf das Gehäuse (2) axial fixiert und um eine erste Achse parallel zu der zentralen Längsachse drehbar ist, wobei das erste Kolbenstangen-Antriebselement (67) betriebsmäßig mit einem zweiten Kupplungsteil (68r) der unidirektionalen Ratschenkupplung (65, 68r) gekoppelt ist, wobei das erste Kolbenstangen-Antriebselement (67) und die erste Kolbenstange (45) drehfest miteinander verbunden sind, sodass eine Drehung des ersten Kolbenstangen-Antriebselements (67) in einer ersten Richtung um die erste Achse mit einer distalen schraubenförmigen Bewegung der ersten Kolbenstange (45) in dem Mutterelement (60) verbunden ist und eine Drehung des ersten Kolbenstangen-Antriebselements (67) in einer zweiten Richtung um die erste Achse mit einer proximalen schraubenförmigen Bewegung der ersten Kolbenstange (45) in dem Mutterelement (60) verbunden ist, und
∘ ein zweites Kolbenstangen-Antriebselement (69), das in Bezug auf das Gehäuse (2) axial fixiert und um eine zweite Achse parallel zu der zentralen Längsachse drehbar ist, wobei das zweite Kolbenstangen-Antriebselement (69) drehfest mit dem ersten Kolbenstangen-Antriebselement (67) gekoppelt ist, wobei das zweite Kolbenstangen-Antriebselement (69) und der hintere Kolbenstangenabschnitt (55) drehfest miteinander verbunden sind, sodass eine Drehung des zweiten Kolbenstangen-Antriebselements (69) in einer ersten Richtung um die zweite Achse mit einer distalen schraubenförmigen Bewegung des hinteren Kolbenstangenabschnitts (55) in dem Mutterelement (60) verbunden ist und eine Drehung des zweiten Kolbenstangen-Antriebselements (69) in einer zweiten Richtung um die zweite Achse mit einer proximalen schraubenförmigen Bewegung des hinteren Kolbenstangenabschnitts (55) in dem Mutterelement (60) verbunden ist,
das Verfahren umfassend:
(i) Anordnen der ersten Kolbenstange (45) und der zweiten Kolbenstange (50) in dem Mutterelement (60), sodass die erste Kontaktfläche (48) distal von einer endgültigen ersten Kontaktflächenmontageposition positioniert ist und die zweite Kontaktfläche (58) distal von einer endgültigen zweiten Kontaktflächenmontageposition positioniert ist,
(ii) Ausrichten des Kartuschenhalters (11) und des Gehäuses (2) entlang der zentralen Längsachse auf eine Weise, dass die erste Kolbenstruktur (25, 29) an der ersten Achse ausgerichtet ist und die zweite Kolbenstruktur (35, 39) an der zweiten Achse ausgerichtet ist,
(iii) Herbeiführen einer konvergierenden relativen axialen Bewegung zwischen dem Kartuschenhalter (11) und dem Gehäuse (2), wobei die Bewegung eine erste Teilbewegung umfasst, die den Kartuschenhalter (11) und das Gehäuse (2) in eine Zwischenmontageposition bringt und während der die erste Kontaktfläche (48) mithilfe der ersten Kolbenstruktur (25, 29) proximal relativ zu dem Mutterelement (60) bewegt wird und die zweite Kontaktfläche (58) proximal relativ zu dem hinteren Kolbenstangenabschnitt (55) bewegt wird, wodurch die zweite Kolbenstange (50) eine Zwischenkonfiguration der zweiten Kolbenstangenanordnung einnimmt, und eine zweite Teilbewegung umfasst, die den Kartuschenhalter (11) und das Gehäuse (2) in eine Endmontageposition bringt und während der die erste Kolbenstange (45), die zweite Kolbenstange (50) und das Mutterelement (60) gemeinsam relativ zu dem ersten Kolbenstangen-Antriebselement (67) bewegt werden, wodurch das erste Kupplungsteil (65) der unidirektionalen Ratschenkupplung (65, 68r) mit dem zweiten Kupplungsteil (68r) der unidirektionalen Ratschenkupplung (65, 68r) in Eingriff kommt, wodurch das erste Kolbenstangen-Antriebselement (67) unidirektional in die erste Richtung um die erste Achse drehbar wird und das zweite Kolbenstangen-Antriebselement (67) unidirektional in die erste Richtung um die zweite Achse drehbar wird,
(iv) axiales Verbinden des vorderen Kolbenstangenabschnitts (51) und des hinteren Kolbenstangenabschnitts (55) in der Zwischenmontagekonfiguration der zweiten Kolbenstangenanordnung, und
(v) axiales Verbinden des Kartuschenhalters (11) und des Gehäuses (2) in der Endmontageposition.

## Revendications

1. Dispositif d'administration de médicament (1) comprenant :
- une unité de cartouche (10) comprenant un porte-cartouche (11) portant une cartouche de médicament (20) ayant un corps de cartouche (21) et une structure de piston (25, 29), et
- une unité d'expulsion de dose comprenant
∘ un logement (2) s'étendant le long d'un axe longitudinal,
∘ une tige de piston (45) pour mettre sous pression la cartouche de médicament (20), la tige de piston (45) comprenant une surface de contact (48) adaptée pour entrer en contact avec la structure de piston (25, 29), et un filetage non autobloquant (46),
∘ un élément d'écrou (60) fixe en rotation par rapport au logement (2) et en prise avec le filetage non autobloquant (46), et
∘ un élément d'entraînement de tige de piston (67) fixé axialement par rapport au logement (2) et pouvant tourner autour de l'axe longitudinal,
dans lequel l'élément d'entraînement de tige de piston (67) et la tige de piston (45) sont enclenchés en rotation, de sorte qu'une rotation de l'élément d'entraînement de tige de piston (67) dans une première direction est associée au mouvement distal de la tige de piston (45) à travers l'élément d'écrou (60), et une rotation de l'élément d'entraînement de tige de piston (67) dans une deuxième direction est associée au mouvement proximal de la tige de piston (45) à travers l'élément d'écrou (60),
**caractérisé en ce que** l'élément d'écrou (60) porte une première partie de couplage (65) d'un couplage à cliquet unidirectionnel (65, 68r), et l'élément d'entraînement de tige de piston (67) est couplé de manière fonctionnelle avec une deuxième partie de couplage (68r) du couplage à cliquet unidirectionnel (65, 68r),
dans lequel, dans un état pré-assemblé, l'élément d'écrou (60) prend une première position d'élément d'écrou par rapport au logement (2) et l'élément d'entraînement de tige de piston (67) dans lequel la première partie de couplage (65) et la deuxième partie de couplage (68r) ne sont plus en prise et dans un état assemblé l'élément d'écrou (60) prend une deuxième position d'élément d'écrou par rapport au logement (2) et l'élément d'entraînement de tige de piston (67) dans lequel la première partie de couplage (65) et la deuxième partie de couplage (68r) sont en prise pour rendre l'élément d'entraînement de tige de piston (67) rotatif de manière unidirectionnelle dans la première direction, et
dans lequel l'élément d'écrou (60) est configuré pour subir un déplacement irréversible de la première position d'élément d'écrou à la deuxième position d'élément d'écrou pendant l'assemblage de l'unité d'expulsion de dose et de l'unité de cartouche (10) en réponse à un mouvement axial relatif convergent entre le logement (2) et le porte-cartouche (11), après que le contact entre la structure de piston (25, 29) et la surface de contact (48) a été établi.

2. Dispositif d'administration de médicament selon la revendication 1, dans lequel le porte-cartouche (11) comprend une portion de rebord proximale (13) qui applique une force dirigée de manière proximale à l'élément d'écrou (60) pendant le mouvement axial relatif convergent entre le logement (2) et le porte-cartouche (11), la force dirigée de manière proximale amenant l'élément d'écrou (60) de la première position d'élément d'écrou à la deuxième position d'élément d'écrou.

3. Dispositif d'administration de médicament selon la revendication 1 ou 2, dans lequel l'unité de cartouche (10) comprend en outre
- une deuxième cartouche de médicament (30) ayant un deuxième corps de cartouche (31) et une deuxième structure de piston (35, 39), la cartouche de médicament (20) et la deuxième cartouche de médicament (30) étant maintenues en parallèle par le porte-cartouche (11),
et l'unité d'expulsion de dose comprend en outre
- une deuxième tige de piston (50) pour mettre sous pression la deuxième cartouche de médicament (30), la deuxième tige de piston (50) comprenant une deuxième surface de contact (58) adaptée pour entrer en contact avec la deuxième structure de piston (35, 39), et un deuxième filetage de tige de piston (52) en prise avec un deuxième filetage de l'élément d'écrou (60), et
- un deuxième élément d'entraînement de tige de piston (69) fixé axialement par rapport au logement (2) et pouvant tourner autour d'un deuxième axe parallèle à l'axe longitudinal, le deuxième élément d'entraînement de tige de piston (69) et la deuxième tige de piston (50) étant enclenchés en rotation, et le deuxième élément d'entraînement de tige de piston (69) étant couplé en rotation avec l'élément d'entraînement de tige de piston (67), et
dans lequel la deuxième tige de piston (50) a une dimension axialement variable qui est établie pendant l'assemblage de l'unité d'expulsion de dose et de l'unité de cartouche (10), après que le contact entre la deuxième structure de piston (35, 39) et la deuxième tige de piston (50) a été établi.

4. Dispositif d'administration de médicament selon la revendication 3, dans lequel la deuxième tige de piston (50) comprend une portion de tige de piston avant (51) et une portion de tige de piston arrière (55) capables de subir un mouvement de coulissement relatif l'une par rapport à l'autre, et dans lequel la dimension axialement variable est établie en enclenchant axialement la portion de tige de piston avant (51) et la portion de tige de piston arrière (55).

5. Dispositif d'administration de médicament selon la revendication 4, dans lequel l'élément d'entraînement de tige de piston (67) et le deuxième élément d'entraînement de tige de piston (69) sont agencés dans un boîtier (70) qui est fixé au logement (2), dans lequel le boîtier (70) est muni d'une fente distale (72) et d'une fente proximale (73) espacées axialement de la fente distale (72), et dans lequel l'élément d'écrou (60) comprend une saillie (64) adaptée pour se déplacer d'une position dans la fente distale (72) à une position dans la fente proximale (73) pendant le déplacement de l'élément d'écrou (60) de la première position d'élément d'écrou à la deuxième position d'élément d'écrou et structurée pour empêcher un déplacement ultérieur de l'élément d'écrou (60) vers la première position d'élément d'écrou.

6. Dispositif d'administration de médicament selon la revendication 4 ou 5, comprenant en outre une roue d'engrenage centrale (68), dans lequel l'élément d'entraînement de tige de piston (67) comprend une première denture de roue d'engrenage (67t) et le deuxième élément d'entraînement de tige de piston (69) comprend une deuxième denture de roue d'engrenage (69t), et dans lequel l'élément d'entraînement de tige de piston (67) et le deuxième élément d'entraînement de tige de piston (69) sont couplés en rotation par l'intermédiaire de la roue d'engrenage centrale (68).

7. Dispositif d'administration de médicament selon la revendication 6, dans lequel la deuxième partie de couplage (68r) fait partie de la roue d'engrenage centrale (68).

8. Dispositif d'administration de médicament selon l'une quelconque des revendications 4 à 7, dans lequel le deuxième filetage de tige de piston (52) est autobloquant.

9. Dispositif d'administration de médicament selon l'une quelconque des revendications 4 à 8, dans lequel la portion de tige de piston avant (51) et la portion de tige de piston arrière (55) sont enclenchées par soudage au laser à travers un alésage (19) dans le porte-cartouche (11).

10. Dispositif d'administration de médicament selon l'une quelconque des revendications 4 à 9, dans lequel la cartouche de médicament (20) a en outre une sortie de médicament scellée par un septum pénétrable (22), et la deuxième cartouche de médicament (30) a en outre une deuxième sortie de médicament scellée par un deuxième septum pénétrable (32), et dans lequel lorsque le septum pénétrable (22) et le deuxième septum pénétrable (32) sont axialement alignés par rapport au porte-cartouche (11), la deuxième structure de piston (35, 39) est positionnée de manière distale à la structure de piston (25, 29).

11. Procédé de réalisation de réglage du point zéro d'un dispositif d'administration de médicament (1) comprenant A) une unité de cartouche (10) comprenant un porte-cartouche (11) portant une cartouche de médicament (20) ayant un corps de cartouche (21) et une structure de piston (25, 29), et B) une unité d'expulsion de dose comprenant un logement (2) s'étendant le long d'un axe longitudinal, une tige de piston (45) pour la mise sous pression de la cartouche de médicament (20), la tige de piston (45) comprenant une surface de contact (48) adaptée pour venir en contact avec la structure de piston (25, 29), et un filetage non autobloquant (46), un élément d'écrou (60) fixe en rotation par rapport au logement (2) et en prise avec le filetage non autobloquant (46), l'élément d'écrou (60) portant une première partie de couplage (65) d'un couplage à cliquet unidirectionnel (65, 68r), et un élément d'entraînement de tige de piston (67) fixé axialement par rapport au logement (2) et pouvant tourner autour de l'axe longitudinal, l'élément d'entraînement de tige de piston (67) étant couplé de manière fonctionnelle avec une deuxième partie de couplage (68r) du couplage à cliquet unidirectionnel (65, 68r), dans lequel l'élément d'entraînement de tige de piston (67) et la tige de piston (45) sont enclenchés en rotation, de telle sorte qu'une rotation de l'élément d'entraînement de tige de piston (67) dans une première direction est associée au mouvement hélicoïdal distal de la tige de piston (45) dans l'élément d'écrou (60), et une rotation de l'élément d'entraînement de tige de piston (67) dans une deuxième direction est associée au mouvement hélicoïdal proximal de la tige de piston (45) dans l'élément d'écrou (60), le procédé comprenant :
(i) l'agencement de la tige de piston (45) dans l'élément d'écrou (60) de telle sorte que la surface de contact (48) est positionnée de manière distale à une position d'assemblage de surface de contact finale,
(ii) la mise en butée mutuelle de la structure de piston (25, 29) et de la surface de contact (48),
(iii) le fait d'induire un mouvement axial relatif convergent entre le porte-cartouche (11) et le logement (2), en forçant ainsi d'abord la tige de piston (45) de manière proximale dans l'élément d'écrou (60) au moyen de la structure de piston (25, 29) et ensuite le déplacement axial de l'élément d'écrou (60) par rapport au logement (2) d'une première position d'élément d'écrou dans laquelle la première partie de couplage (65) et la deuxième partie de couplage (68r) ne sont plus en prise vers une deuxième position d'élément d'écrou dans laquelle la première partie de couplage (65) et la deuxième partie de couplage (68r) sont en prise, le fait de rendre l'élément d'entraînement de tige de piston (67) rotatif de manière unidirectionnelle dans la première direction, et
(iv) l'enclenchement de manière axiale du porte-cartouche (11) et du logement (2).

12. Procédé selon la revendication 11, dans lequel la mise en butée mutuelle de la structure de piston (25, 29) et de la surface de contact (48) implique le fait d'induire un mouvement axial relatif convergent entre le porte-cartouche (11) et le logement (2).

13. Procédé de réalisation d'un réglage de point zéro d'un dispositif d'administration de médicament (1) comprenant
- une unité de cartouche (10) comprenant un porte-cartouche (11) portant
∘ une première cartouche de médicament (20) ayant un premier corps de cartouche (21) et une première structure de piston (25, 29), et
∘ une deuxième cartouche de médicament (30) ayant un deuxième corps de cartouche (31) et une deuxième structure de piston (35, 39), et
- une unité d'expulsion de dose comprenant
∘ un logement (2) s'étendant le long d'un axe central longitudinal,
∘ une première tige de piston (45) pour mettre sous pression la première cartouche de médicament (20), la première tige de piston (45) comprenant une première surface de contact (48) adaptée pour entrer en contact avec la première structure de piston (25, 29), et un filetage non autobloquant (46),
∘ une deuxième tige de piston (50) pour mettre sous pression la deuxième cartouche de médicament (30), la deuxième tige de piston (50) comprenant une portion de tige de piston avant (51) ayant une deuxième surface de contact (58) adaptée pour entrer en contact avec la deuxième structure de piston (35, 39), et une portion de tige arrière (55) ayant un deuxième filetage de tige de piston (52), la portion de tige de piston avant (51) et la portion de tige arrière (55) étant capables de subir un mouvement de coulissement relatif l'une par rapport à l'autre,
∘ un élément d'écrou (60) fixé en rotation par rapport au logement (2) et respectivement en prise avec le filetage non autobloquant (46) et le deuxième filetage de tige de piston (52), l'élément d'écrou (60) portant une première partie de couplage (65) d'un couplage à cliquet unidirectionnel (65, 68r),
∘ un premier élément d'entraînement de tige de piston (67) fixé axialement par rapport au logement (2) et pouvant tourner autour d'un premier axe parallèle à l'axe central longitudinal, le premier élément d'entraînement de tige de piston (67) étant couplé de manière fonctionnelle avec une deuxième partie de couplage (68r) du couplage à cliquet unidirectionnel (65, 68r), et le premier élément d'entraînement de tige de piston (67) et la première tige de piston (45) étant enclenchés en rotation de telle sorte qu'une rotation du premier élément d'entraînement de tige de piston (67) dans une première direction autour du premier axe est associée au mouvement hélicoïdal distal de la première tige de piston (45) dans l'élément d'écrou (60), et une rotation du premier élément d'entraînement de tige de piston (67) dans une deuxième direction autour du premier axe est associée au mouvement hélicoïdal proximal de la première tige de piston (45) dans l'élément d'écrou (60), et
∘ un deuxième élément d'entraînement de tige de piston (69) fixé axialement par rapport au logement (2) et pouvant tourner autour d'un deuxième axe parallèle à l'axe central longitudinal, le deuxième élément d'entraînement de tige de piston (69) étant couplé en rotation avec le premier élément d'entraînement de tige de piston (67), et le deuxième élément d'entraînement de tige de piston (69) et la portion de tige de piston arrière (55) étant enclenchés en rotation de sorte qu'une rotation du deuxième élément d'entraînement de tige de piston (69) dans une première direction autour du deuxième axe est associée au mouvement hélicoïdal distal de la portion de tige de piston arrière (55) dans l'élément d'écrou (60), et une rotation du deuxième élément d'entraînement de tige de piston (69) dans une deuxième direction autour du deuxième axe est associée au mouvement hélicoïdal proximal de la portion de tige de piston arrière (55) dans l'élément d'écrou (60),
le procédé comprenant :
(i) l'agencement de la première tige de piston (45) et de la deuxième tige de piston (50) dans l'élément d'écrou (60) de telle sorte que la première surface de contact (48) est positionnée de manière distale à une première position d'assemblage de surface de contact finale et la deuxième surface de contact (58) est positionnée de manière distale à une deuxième position d'assemblage de surface de contact finale,
(ii) l'alignement du porte-cartouche (11) et du logement (2) le long de l'axe central longitudinal de sorte que la première structure de piston (25, 29) est alignée avec le premier axe et la deuxième structure de piston (35, 39) est alignée avec le deuxième axe,
(iii) le fait d'induire un mouvement axial relatif convergent entre le porte-cartouche (11) et le logement (2), ledit mouvement comprenant un premier mouvement partiel qui amène le porte-cartouche (11) et le logement (2) à une position d'assemblage intermédiaire et au cours duquel la première surface de contact (48) est déplacée de manière proximale par rapport à l'élément d'écrou (60) au moyen de la première structure de piston (25, 29) et la deuxième surface de contact (58) est déplacée de manière proximale par rapport à la portion de tige de piston arrière (55), la deuxième tige de piston (50) obtenant ainsi une configuration d'assemblage intermédiaire de deuxième tige de piston, et un deuxième mouvement partiel qui amène le porte-cartouche (11) et le logement (2) à une position d'assemblage finale et au cours de laquelle la première tige de piston (45), la deuxième tige de piston (50), et l'élément d'écrou (60) sont déplacés conjointement par rapport au premier élément d'entraînement de tige de piston (67), la première partie de couplage (65) du couplage à cliquet unidirectionnel (65, 68r) entrant ainsi en prise avec la deuxième partie de couplage (68r) du couplage à cliquet unidirectionnel (65, 68r), le fait de rendre le premier élément d'entraînement de tige de piston (67) rotatif de manière unidirectionnelle dans la première direction autour du premier axe et le deuxième élément d'entraînement de tige de piston (67) rotatif de manière unidirectionnelle dans la première direction autour du deuxième axe,
(iv) l'enclenchement axial de la portion de tige de piston avant (51) et de la portion de tige de piston arrière (55) dans la configuration intermédiaire d'assemblage de deuxième tige de piston, et
(v) l'enclenchement axial du porte-cartouche (11) et du logement (2) dans la position d'assemblage finale.
